# EUROPEAN PATENT APPLICATION

(11) **EP 2 394 595 A1**
(43) Date of publication of application: **14.12.2011**
(21) Application number: 10738427.3
(22) Date of filing: 25.01.2010
(51) Int. Cl.: A61B 18/14, A61B 1/00, A61B 17/221

(54) **MANIPULATION INSTRUMENT**

(30) Priority: 06.02.2009 JP 2009026583
(71) Applicant: OLYMPUS MEDICAL SYSTEMS CORP., Tokyo 151-0072 (JP)
(72) Inventor: YANUMA, Yutaka, Tokyo 151-0072 (JP); FUJII, Hideki, Tokyo 151-0072 (JP)
(74) Representative: Schicker, Silvia
(86) International application number: PCT/JP2010/050889
(87) International publication number: WO 2010/090089

(57) **Abstract**

This treatment instrument includes: a flexible wire (50) that has a first end part (51) and a second end part (52), and that has a treatment part (3) at an intermediate part (53) for conducting treatment within a body cavity; small-diameter parts (51a, 52a) where at least a portion of the outer circumferential face of the wire has a smaller diameter or is denuded relative to the rest; a bundled part (55) where the wire is bundled so as to at least include the small-diameter parts; and a manipulation wire (5) that is connected to this bundled part.

## Description

### BACKGROUND OF THE INVENTION

### [Field of the Invention]

The present invention relates to a treatment instrument for treating body tissue.
This application claims priority on Japanese Patent Application No. 2009-026583 filed on February 6, 2009, the content of which is incorporated herein by reference.

### [Description of Related Art]

Conventionally, as treatment instruments for conducting treatment of body tissue, there are known to be lithotriptic baskets that tightly bind and crush calculi with wire, and high-frequency treatment instruments that grasp body tissue and that perform cautery incisions and the like with wire capable of carrying high-frequency current.

As examples of such treatment instruments, there is, for example, the endoscopic treatment instrument described in Patent Document 1 which is provided with a wire capable of carrying high-frequency current. This endoscopic treatment instrument is provided with a manipulation wire that passes through a sheath, a connecting pipe that is provided on the distal end side of this manipulation wire, and a loop wire (wire) of which both ends are inserted into and fixed to this connecting pipe.
According to the endoscopic treatment instrument described in Patent Document 1, it is possible to securely grasp body tissue with this loop wire (wire), and excise body tissue such as polyps and the like.

[Patent Document 1] Japanese Unexamined Patent Application, First Publication No. 2000-271146.

### SUMMARY OF THE INVENTION

### [Problem to be Solved by the Invention]

However, with the endoscopic treatment instrument described in Patent Document 1, both ends of the wire are inserted together into and fixed to the connecting pipe. Consequently, there is the problem that it is necessary to configure the inner diameter of the connecting pipe to be at least twice the outer diameter of the wire, and that it is difficult to reduce the diameter of the connecting pipe. Moreover, when the connecting pipe that bulges in the radial direction relative to the wire undergoes sliding movement relative to that sheath, sliding resistance occurs. Consequently, there is the problem that the tractive force and pressing force that are required to operate the manipulation wire are large.

The present invention was made in light of the foregoing circumstances, and its object is to provide a treatment instrument that enables reduced diameter.

### [Means for Solving the Problem]

In order to solve the aforementioned problems, the present invention is provided with the following structure.
The treatment instrument of the present invention is provided with: a flexible wire that has a first end part and a second end part, and that has a treatment part at an intermediate part for conducting treatment within a body cavity; small-diameter parts where at least a portion of the outer circumferential face of the wire has a smaller diameter or is denuded relative to the rest; a bundled part where the wire is bundled so as to at least include the small-diameter parts; and a manipulation wire that is connected to the bundled part.

According to the present invention, a small-diameter part of the wire has a portion where the diameter is smaller than that of the other portion of the wire. Accordingly, the bundled part where the wire is bundled so as to include the small-diameter parts is more compact than in the case where the same number of wires is bundled without inclusion of small-diameter parts. Accordingly, it is possible to reduce the maximum external diameter of the connecting part of the bundled part and the manipulation wire, and create a treatment instrument of smaller diameter.

With respect to the treatment instrument of the present invention, it is preferable that the small-diameter parts be provided at least at the first end part or the second end part.
In this case, as the small-diameter parts are provided at the end parts, it is possible to impart a larger diameter than the small-diameter parts to the intermediate part that serves to conduct treatment within the body cavity.

It is also preferable that the treatment instrument of the present invention has a connecting tube into which at least the small-diameter parts and the manipulation wire are inserted, and in which they are fixed.
In this case, as the small-diameter parts and manipulation wire are together inserted into and fixed to a connecting tube, the respective external faces of the small-diameter parts and the manipulation wire are supported on the inner circumferential face of the connecting tube. Accordingly, fixation can be conducted simply and reliably compared to the case where the respective end parts of the small-diameter parts and the manipulation wire are joined together.

The treatment instrument of the present invention is provided with: a flexible wire that has a first end part and a second end part, and that has a treatment part at an intermediate part for conducting treatment within a body cavity; a small-diameter part where at least a portion of the outer circumferential face of the first end part has a smaller diameter or is denuded relative to the outer circumferential face of the intermediate part of the wire; and a bundled part where the wire is curved or folded back, and the small-diameter part is fixed to the outer circumferential face of the wire. The length from the treatment part to the second end part is longer than the length from the treatment part to the first end part.
According to the present invention, as the small-diameter part is bundled and fixed to the outer circumferential face of the wire, it is possible to reduce its maximum external diameter compared to the case where a conventional first end part that is not of small diameter is fixed to the outer circumferential face of the wire.

With respect to the treatment instrument of the present invention, it is also acceptable for the small-diameter parts to be tapered so that diameter narrows in a tapered form.
In this case, as the external diameter of the wire in the tapered portion gradually changes, a bump in the outer circumferential face of the wire is eliminated. Consequently, it is possible to prevent damage resulting from wire breakage or the like due to catching of this bump on an object or the like.

In addition, with respect to the treatment instrument of the present invention, it is preferable that the wire has electrical conductivity, and that it be further provided with conductive members that are electrically connected to the aforementioned wire and that serve to supply high-frequency current.
In this case, it is possible to conduct high-frequency current from the conductive members via the wire to a target object that contacts the wire.

Moreover, with respect to the treatment instrument of the present invention, it is preferable that the manipulation wire has electrical conductivity.
The configuration can be simplified in this case, because it is possible to conduct high-frequency current to the wire and to manipulate the wire by means of the manipulation wire.

With respect to the treatment instrument of the present invention, it is also preferable that the treatment part be a snare wire that tightly binds body tissue and performs cautery cutting.
In this case, the small-diameter part has a smaller diameter relative to the snare loop part in the snare wire that contacts body tissue in order to tightly bind the body tissue. Accordingly, it is possible to reduce the diameter of the bundled part regardless of the thickness of the snare loop part.

It is also acceptable for the treatment instrument of the present invention to have a plurality of the wires, and for the treatment part to be provided with a basket that contacts the body tissue at three or more separate points relative to a substance within the body cavity, and that captures the substance within the body cavity.

### [Effects of the Invention]

According to the treatment instrument of the present invention, small-diameter parts are provided at portions of the wires. Consequently, it is possible to reduce maximum external diameter of a bundled part where the wires are bundled so as to include more than one small-diameter part. Thereby it becomes to reduce the diameter of the treatment instrument provided with the wires.

### BRIEF DESCRIPTION OF THE DRAWINGS

Fig. 1 is a plan view which shows a treatment instrument of a first embodiment of the present invention.
Fig. 2 is a plan view which shows a treatment part of the same treatment instrument.
Fig. 3 is a plan view which shows a wire of the same treatment instrument.
Fig. 4 is a side view which shows operations during use of the same treatment instrument in a partial cross-section.
Fig. 5 is a side view which shows operations during use of the same treatment instrument in a partial cross-section.
Fig. 6A is a drawing which shows the configuration of a variation of the same treatment instrument.
Fig. 6B is a drawing which shows the configuration of a variation of the same treatment instrument.
Fig. 6C is a drawing which shows the configuration of a variation of the same treatment instrument.
Fig. 6D is a drawing which shows the configuration of a variation of the same treatment instrument.
Fig. 7A is a drawing which shows another variation of the same treatment instrument.
Fig. 7B is a drawing which shows another variation of the same treatment instrument.
Fig. 7C is a drawing which shows another variation of the same treatment instrument.
Fig. 8A is a side view which shows a partial configuration of a treatment instrument of a second embodiment of the present invention.
Fig. 8B is a side view which shows a partial configuration of the treatment instrument of the second embodiment of the present invention.
Fig. 8C is a side view which shows a partial configuration of the treatment instrument of the second embodiment of the present invention.
Fig. 8D is a side view which shows a partial configuration of the treatment instrument of the second embodiment of the present invention.
Fig. 9A is a side view which shows a partial configuration of a treatment instrument of a third embodiment of the present invention in a partial cross-section.
Fig. 9B is a side view which shows a partial configuration of the treatment instrument of the third embodiment of the present invention in a partial cross-section.
Fig. 10A is a side view which shows a partial configuration of the treatment instrument of a fourth embodiment of the present invention in a partial cross-section.
Fig. 10B is a side view which shows a partial configuration of the treatment instrument of the fourth embodiment of the present invention in a partial cross-section.
Fig. 11A is a side view which shows a partial configuration of the treatment instrument of a fifth embodiment of the present invention in a partial cross-section.
Fig. 11B is a side view which shows a partial configuration of the treatment instrument of the fifth embodiment of the present invention in a partial cross-section.

### PREFERRED EMBODIMENTS

### (First embodiment)

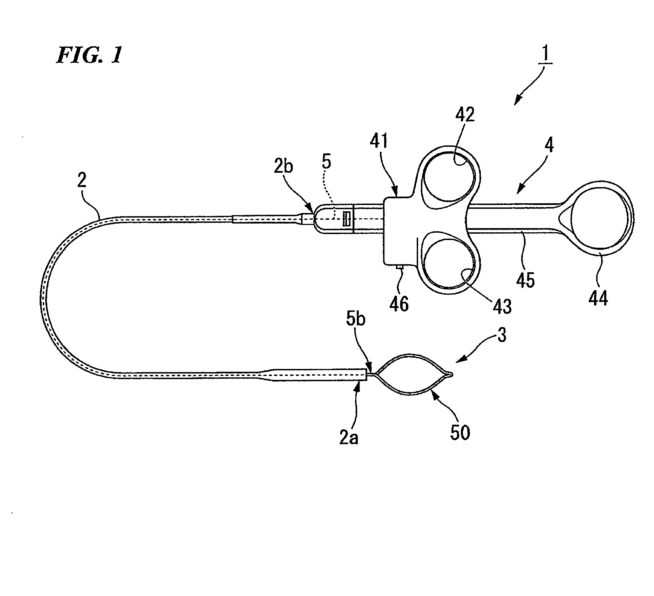

A treatment instrument of a first embodiment of the present invention is described below with reference to Fig. 1 to Fig. 3.
Fig. 1 is a plan view which shows a treatment instrument 1 of the present embodiment. As shown in Fig. 1, the treatment instrument 1 is provided with a treatment part 3 that conducts treatment within a body cavity, a manipulation part 4 that is disposed at the side near the operator and that serves to manipulate the treatment part 3, and a tubular sheath 2 that is disposed between the treatment part 3 and the manipulation part 4.
A manipulation wire 5 that is composed of flexible metallic wire and that is respectively connected to the treatment part 3 and manipulation part 4 runs through the interior of the sheath 2.

With respect to the manipulation part 4, one may appropriately adopt a configuration of a conventional manipulation part of a treatment instrument. For example, one may adopt a manipulation part provided with a manipulation body 45 into which the manipulation wire 5 is inserted so as to be capable of freely moving forward or backward and which extends in the axial direction, a slider 41 that is capable of freely moving forward or backward relative to the manipulation body 45 and that serves to conduct forward/backward operation of the manipulation wire 5 relative to the sheath 2, and a supply electrode 46 that is provided in this slider 41 and that serves to connect the manipulation wire 5 to an external high-frequency power supply device (not illustrated in the drawings). High-frequency current can be carried from the high-frequency power supply device to the supply electrode 46, manipulation wire 5, and a wire 50 which are conductive members that have electrical conductivity.
With respect to this type of manipulation part 4, in order to optimally conduct forward and backward operation of the slider 41 relative to the manipulation body 45, it is preferable to provide finger grips 42, 43, and 44 that may be gripped by the fingers of the operator.

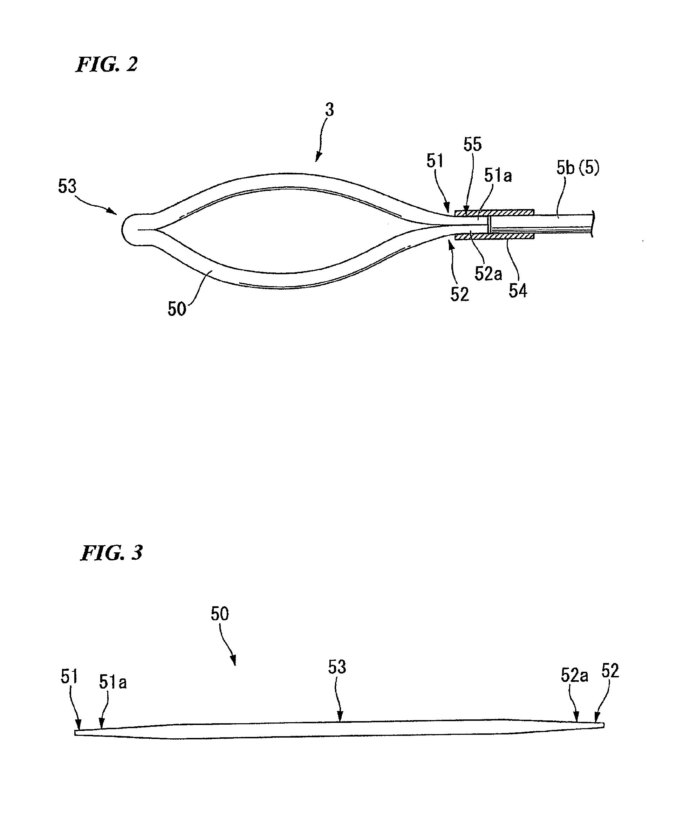

Fig. 2 is a plan view which shows a partial cross-section of the treatment part 3. As shown in Fig. 2, the treatment part 3 of the present embodiment is a snare wire consisting of a wire 50. With respect to the wire 50, a first end part 51 and a second end part 52 are adjacently disposed with the same orientation and are inserted into a connecting tube 54. Accordingly, the wire 50 constitutes a snare loop of closed annular shape. Moreover, in the present embodiment, the first end part 51 and second end part 52 respectively have small-diameter parts 51a and 52a that are configured with a smaller diameter than an intermediate part 53.

Furthermore, a bundled part 55 where the wire 50 is bundled is configured by the first end part 51, second end part 52, and connecting tube 54. In addition, a distal end 5b of the manipulation wire 5 inserts into the connecting tube 54 so as to face the first end part 51 and second end part 52.

The wire 50 and manipulation wire 5 are both inserted into the connecting tube 54, and are fixed thereto by, for example, brazing. With respect to the method of connecting the manipulation wire 5 and the wire 50, so long as it is a method that uses the connecting tube 54, an appropriate method other than brazing may be adopted such as welding, soldering, or caulking.

Fig. 3 is a plan view which shows the wire 50. As shown in Fig. 3, the wire 50 is a single wire that is composed of metal and that has a first end part 51 and second end part 52. With respect to this wire 50, compared to the intermediate part 53, the first end part 51 and second end part 52 have small-diameter parts 51a and 52a by forming tapered parts where the diameter narrows in a tapered manner.

With respect to the method of formation of the small-diameter parts 51a and 52a, one may adopt a method where the outer circumferential face of the wire is pared away by centerless taper processing, a method where the end parts of the wire 50 are immersed in a chemical solution to corrode the end parts of the wire 50, and so on. Moreover, although not illustrated in the drawings, it is also possible to form an inclined end face and form the taper by cutting the wire 50 at the first end part 51 or second end part 52 at an angle that is inclined relative to the axial line of the wire 50.

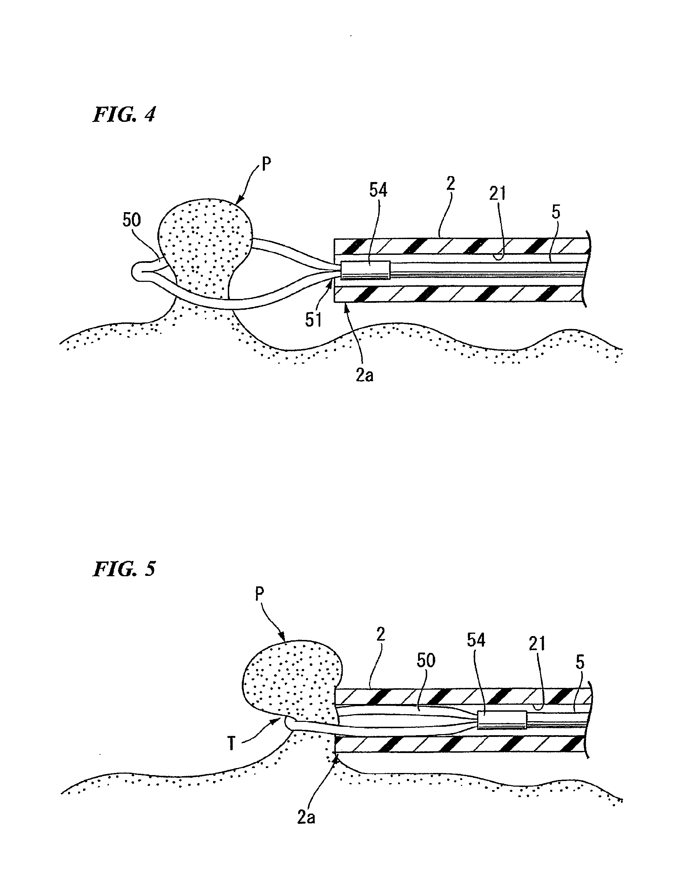

Operations during use of the treatment instrument 1 of the present embodiment having the configuration described above are described with reference to Fig. 4 and Fig. 5.
First, the user inserts a prescribed endoscope into a body cavity of a patient from a natural orifice such as the anus or mouth. In the present embodiment, the endoscope is provided with a guide means capable of guiding the treatment instrument 1 along the endoscope, such as a treatment instrument channel through which the treatment instrument 1 is capable of freely passing with forward or backward movement, and an imaging mechanism capable of imaging the body tissue that is the object of treatment, such as a solid-state imaging device or a fiberscope.
The user guides the distal end of the endoscope to a prescribed position within the body cavity of the patient, and confirms the body tissue that is the object of treatment within the visual field of the imaging mechanism. Next, the user passes the treatment instrument 1 along, for example, the aforementioned treatment instrument channel, and directs it to the interior of the body cavity of the patient through the treatment instrument channel.

Fig. 4 is a side view which shows one process during use of the treatment instrument 1 in a partial cross-section, in a state where the treatment instrument 1 has been guided to the interior of the body cavity. As shown in Fig. 4, the user inserts the treatment instrument 1 into the body cavity from the distal end 2a side of the sheath 2. The user then guides the distal end 2a of the sheath 2 to the vicinity of, for example, a polyp P which is the body tissue that is the object of treatment.

Next, the user causes sliding movement of the slider 41 of the manipulation part 4 (see Fig. 1) relative to the manipulation body 45, and causes pushing movement of the manipulation wire 5 toward the distal end 2a side of the sheath 2, whereupon the wire 50 of the manipulation part 4 that is fixed to the distal end of the manipulation wire 5 projects from the distal end 2a of the sheath 2.

The wire 50 that projects from the distal end 2a of the sheath 2 restores itself to an open state due to its own elasticity, and becomes approximately annular in shape. The user adjusts the position of the wire 50 so that the wire 50 is placed around the polyp P.

Fig. 5 is a side view which shows one process where the polyp P is treated with the wire 50 in a partial cross-section. The user causes sliding movement of the slider 41 of the manipulation part 4 (see Fig. 1) in the reverse of what was described above, toward the base end side (finger grip 44 side) of the manipulation body 45, and the manipulation wire 5 is pulled toward the manipulation part 4 side.
When this is done, the wire 50 and the connecting tube 54 that are fixed to the distal end of the manipulation wire 5 are moved so as to be stored within a lumen 21 of the sheath 2. At this time, as the polyp P is enclosed by the wire 50, the wire 50 contacts the outer face of the polyp P. Furthermore, when the wire 50 is pulled back into the lumen 21 of the sheath 2, the polyp P contacts the distal end 2a of the sheath 2. In this state, the polyp P is tightly bind by both the wire 50 and the sheath 2.

Next, the user causes high-frequency current to be conducted to the treatment part 3 through the supply electrode 46. The high-frequency current is conducted to the supply electrode 46, manipulation wire 5, and wire 50 which are conductive members having electrical conductivity. A description is omitted with respect to other conductive members that serve to electrically connect the respective supply electrode 46, manipulation wire 5, and wire 50.

At a contact portion T of the wire 50 and the polyp P, Joule heat is generated by the high-frequency current, whereupon a cautery incision of body tissue is made at the contact portion T, and the polyp P is excised.
When the polyp P is excised, the polyp P is taken out of the body after excision using a known tissue recovery device that is not illustrated in the drawings, the treatment instrument 1 is withdrawn from the interior of the body cavity, and the treatment sequence is terminated.

With respect to conventional treatment instruments, it was required to reduce the external diameter of the sheath, but it was difficult to reduce diameter, because the maximum external diameter of the member that includes the wire, connecting tube and manipulation wire, and that moves backward and forward through the interior of the sheath is determined by the external diameter of the end part of the wire and the external diameter of the connecting tube. As a result, previously, the connecting tube would slide against the lumen of the sheath at the connecting portion of the wire and the manipulation wire, and resistance would be large when the operator manipulated the manipulation part.

According to the treatment instrument 1 of the present embodiment, a bundled part 55 is configured by bundling and inserting the first end part 51 and second end part 52 which are small-diameter parts into the interior of the connecting tube 54. The total diameter of the first end part 51 and second end part 52 is configured to be more than two times smaller than the diameter of the intermediate part 53. Consequently, the inner diameter of the connecting tube is smaller than the inner diameter of a conventional connecting tube. As a result, even in the case where the thickness of the tube wall of the connecting tube is made the same in order to ensure the strength of the connecting tube, the external diameter of the connecting tube is reduced. Accordingly, with respect to the treatment instrument 1 of the present embodiment, it is possible to optimally conduct forward/backward operation of the treatment part 3 and the manipulation wire 5 within the sheath 2, because the sliding resistance between the lumen 21 and the connecting tube 54 can be reduced in comparison to conventional configurations.
Moreover, it is also possible to compact the interstice of the lumen 21 and connecting tube 54, and further reduce the diameter of the sheath 2.

In the present embodiment, the intermediate part 53 has a larger diameter than the small-diameter parts 51a and 52a. This is because it is necessary to have a thickness that suitably inhibits the wire 50 from excessively cutting into body tissue or the like and severing the body tissue when the intermediate part 53 tightly binds the body tissue. With respect to the wire 50 of the present embodiment, the small-diameter parts 51a and 52a are provided so as to avoid the region where contact is made with body tissue when the body tissue is tightly bind. Accordingly, body tissue such as the polyp P can be cut while cauterizing by applying high-frequency current. Consequently, the wire 50 of the present embodiment is able to combine the performance of a snare wire and reduced sheath diameter in the manner described above.

### (Variation 1)

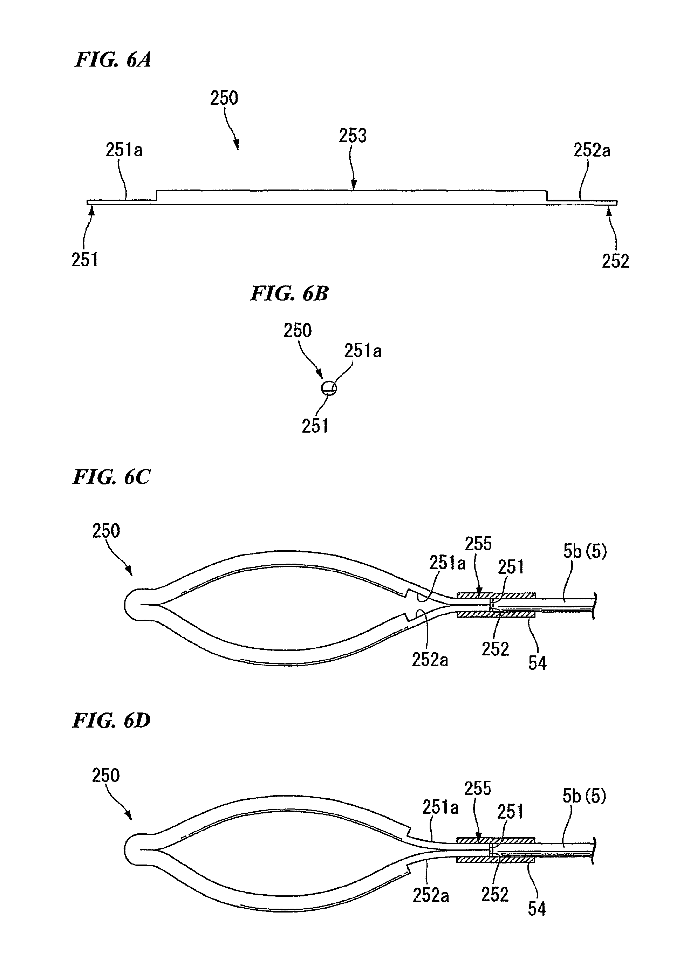

A variation of the wire 50 is described below with reference to Fig. 6A, Fig. 6B, Fig. 6C, and Fig. 6D.
The drawings show a wire 250 of the present variation. Fig. 6A is a side view of the wire 250; Fig. 6B is a frontal view of the wire 250; and Fig. 6C is a sectional view which shows the configuration of the wire 250 after deployment. In addition, Fig. 6D is a sectional view which shows another configuration of the wire 250 after deployment.

As shown in Fig. 6A and Fig. 6B, the wire 250 has a first end part 251 and a second end part 252. The first end part 251 and second end part 252 respectively have small-diameter parts 251a and 252a that are formed by partially denuding the external circumferential face of the wire 250.

As shown in Fig. 6C, the wire 250 has a bundled part 255 that is formed by inserting both the first end part 251 and second end part 252 into the connecting tube 54, and fixing them to the connecting tube 54. Maximum outer diameter when the first end part 251 and second end part 252 are brought together at this time is approximately equal to the outer diameter of the manipulation wire 5.

As shown in Fig. 6D, it is also acceptable to adopt a form that imposes a positional relation where the respective bumps that occur in the small-diameter parts 251a and 252a face mutually outward, when the wire 250 is deployed.

In the present variation, when the first end part 251 and second end part 252 are inserted into the connecting tube 54, the element that regulates the inner diameter of the connecting tube 54 is the outer diameter of the manipulation wire 5. Accordingly, with respect to the present variation, it is possible to establish the inner diameter of the connecting tube 54 at the minimum inner diameter that satisfies the form that is required by the manipulation wire 5 when the treatment part 3 is moved forward or backward. Consequently, the present variation enables minimization of the inner diameter and external diameter of the connecting tube 54. Accordingly, the present variation enables the diameter of the sheath to be reduced like the treatment instrument 1 described above.

### (Variation 2)

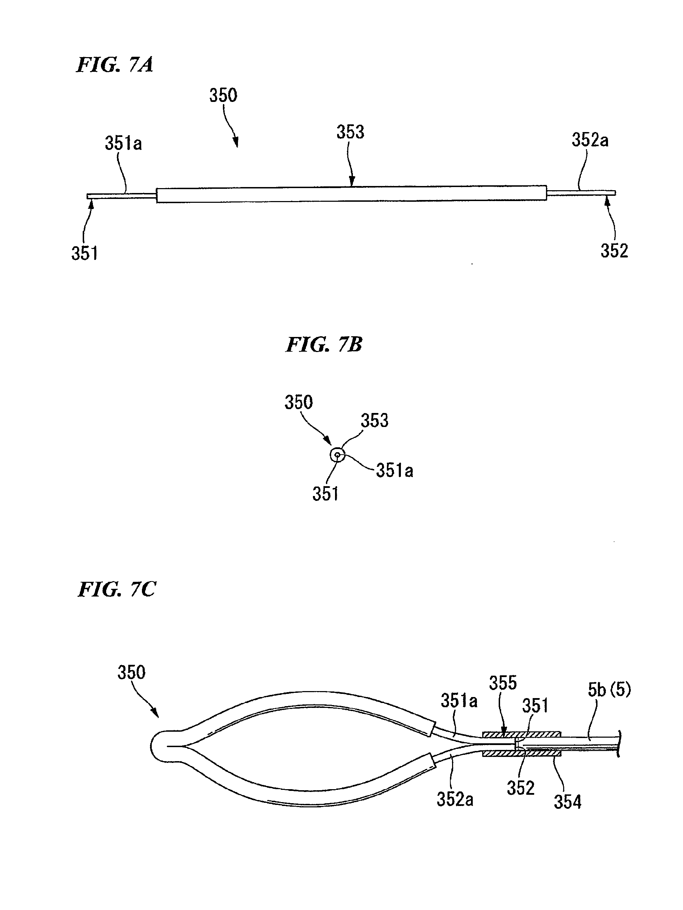

A variation of the wire 50 is described below with reference to Fig. 7A, Fig. 7B, and Fig. 7C.
These drawings show a wire 350 of the present variation. Fig. 7A is a side view of the wire 350; Fig. 7B is a frontal view of the wire 350; and Fig. 7C is a sectional view which shows the configuration of the wire 350 after deployment.

As shown in Fig. 7A and Fig. 7B, the wire 350 of the present variation has a first end part 351 and a second end part 352. The first end part 351 and second end part 352 respectively have small-diameter parts 351a and 352a which are denuded to a prescribed diameter only and reduced diameter relative to an intermediate part 353.
As shown in Fig. 7C, the wire 350 has a bundled part 355 formed by inserting both the first end part 351 and second end part 352 into the connecting tube 54, and fixing them to the connecting tube 54. Maximum outer diameter when the first end part 351 and second end part 352 are brought together at this time is approximately equal to the outer diameter of the manipulation wire 5.
As the diameter of the bundled part can be reduced in the manner described above even with the aforementioned shape, it is possible to obtain the same effect as the treatment instrument 1 described above.

### (Second embodiment)

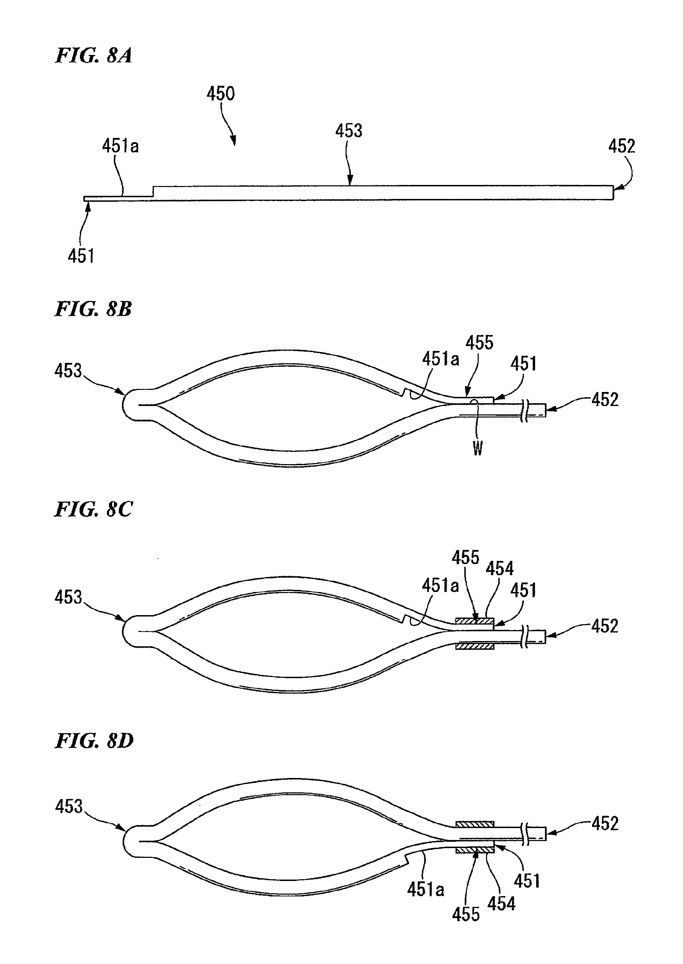

Next, a treatment instrument of a second embodiment of the present invention is described with reference to Fig. 8A to Fig. 8D. In each of the embodiments described below, the same reference symbols are given to items that are common to the configuration of the treatment instrument of the above-described first embodiment, and description thereof is omitted.
The treatment instrument of the present embodiment is not provided with the manipulation wire 5, and is provided with a wire 450 instead of the wire 50.

Fig. 8A is a side view of the wire 450. As shown in Fig. 8A, the wire 450 has a first end part 451 and a second end part 452. The first end part 451 is provided with a small-diameter part 451a that is formed like the small-diameter part 251a.

Fig. 8B is a side view which shows the configuration of the wire 450 after deployment. As shown in Fig. 8B, the wire 450 folds back at an intermediate part 453. Furthermore, a bundled part 455 is formed by welding anchoring the small-diameter part 451a to a connection portion W of the outer face of the wire 450. From the connection portion W to its second end part 452, the wire 450 extends until the manipulation part 4 (see Fig. 1) like the manipulation wire 5 of the first embodiment, and is moved forward or backward by the slider 41.

Fig. 8C is a side view which shows a partial cross-section of another configuration after deployment of the wire 450. As shown in Fig. 8C, the connection portion of the small-diameter part 451a and the outer face of the wire 450 may be inserted and be fixed to a tubular connecting tube 454. Even with this type of configuration, it is possible to obtain the effect of enabling a reduction in diameter of the sheath as in the above-described configurations. Moreover, as the manipulation wire 5 is not provided, and as the second end part 452 side of the wire 450 is connected to the manipulation part 4, the number of components can be reduced.
Fig. 8D is a side view which shows a partial cross-section of another configuration after deployment of the wire 450. As shown in Fig. 8D, the bump that occurs in the small-diameter part 451a may be configured so as to face toward the outside of the snare loop created by the wire 450.

### (Third embodiment)

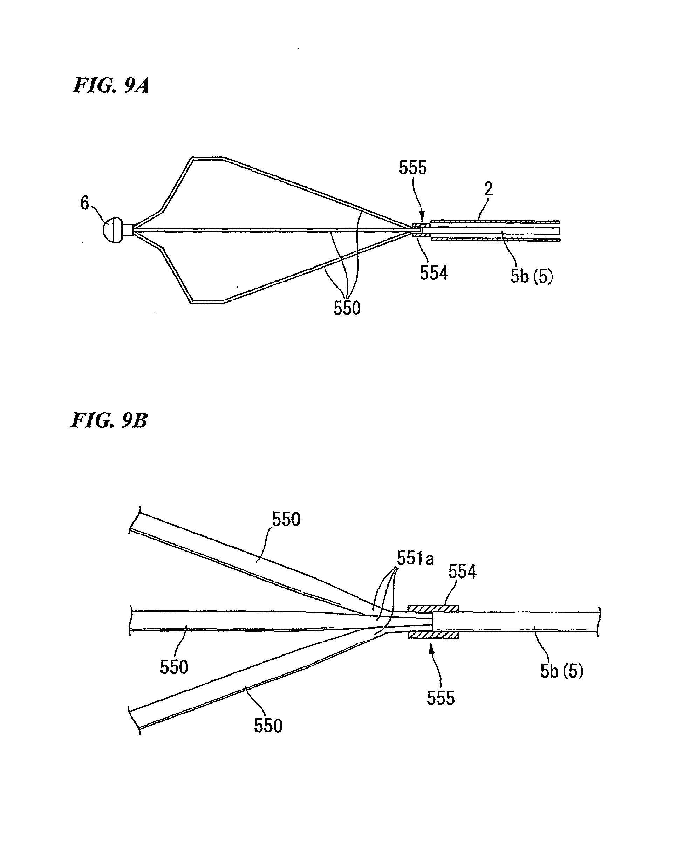

Next, a treatment instrument of a third embodiment of the present invention is described with reference to Fig. 9A and Fig. 9B. In the present embodiment, wires 550 are multiply provided in which small-diameter parts are formed similar to those of the wire 50 of the first embodiment. For example, Fig. 9B is an enlarged view which shows a partial cross-section in the vicinity of a second bundled part 555. As shown in Fig. 9B, small-diameter parts 551a provided at the end parts of the wires 550 are inserted into and fixed to a connecting tube 554. The small-diameter parts 551a are configured by forming tapered parts at both ends of the wires 550. Although not illustrated in the drawings, end parts of the wires 550 including small-diameter parts are also similarly inserted into and fixed to a first bundled part 6.

As shown in Fig. 9A and Fig. 9B, in the present embodiment, the wires 550 are bundled in threes to form the first bundled part 6 and second bundled part 555. Accordingly, an approximately spherical space is formed by the three wires 555. That is, in the present embodiment, the treatment part that has the wires 550 bundled by the first bundled part 6 and second bundled part 555 is used as a so-called basket that captures an object such as body tissue and the like.
This type of basket configuration may be suitably employed, for example, for lithotriptic baskets that serve to crush gallstones and the like, stone extraction baskets that serve to recover gallstones and the like, or basket-type microsnares that serve to crush blood clots and the like.
With respect to such baskets having bundled parts including one or more small-diameter parts, multiple wires are bundled together, with the result that it is possible to reduce the maximum external diameter of the portions where the wires are bundled in comparison to conventional baskets where the bundled portions are of large diameter.

### (Fourth embodiment)

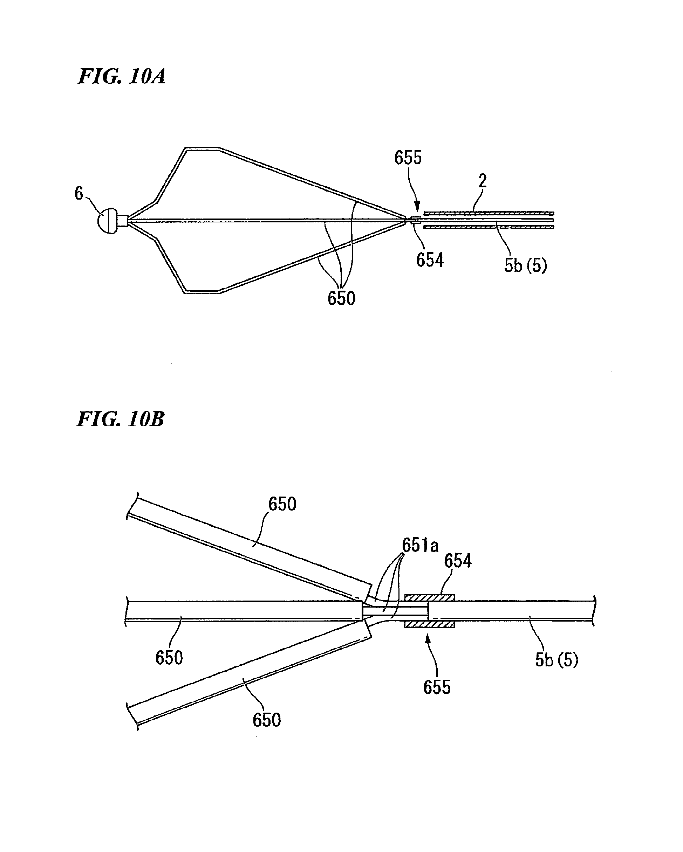

Next, a treatment instrument of a fourth embodiment of the present invention is described with reference to Fig. 10A and Fig. 10B. In the present embodiment, wires 650 are multiply provided in which small-diameter parts are formed similar to those of the wire 250 of the first embodiment. As shown in Fig. 10A, the end parts of the multiple wires 650 are respectively bundled at a first bundled part 6 and a second bundled part 655.
For example, Fig. 10B is an enlarged view which shows a partial cross-section in the vicinity of the second bundled part 655. As shown in Fig. 10B, small-diameter parts 651a provided at the end parts of the wires 650 are inserted into and fixed to a connecting tube 654. The small-diameter parts 651a are configured by denuding a portion of the outer circumferential face at both ends of the wires 650. Although the details are not illustrated in the drawings, end parts of the wires including small-diameter parts are also similarly inserted into and fixed to the first bundled part 6.
A basket configuration similar to that of the third embodiment is obtainable with this type of configuration as well. By bundling multiple wires in the same way as the third embodiment, it is possible to reduce the maximum external diameter of the portions where the wires are bundled in comparison to conventional baskets where the bundled portions are of large diameter.
In the present embodiment, it is also possible to adopt configurations where the orientation of the bumps that occur in the small-diameter parts are changed, in a manner similar to the positional relations shown in Fig. 6C and Fig. 6D.

### (Fifth embodiment)

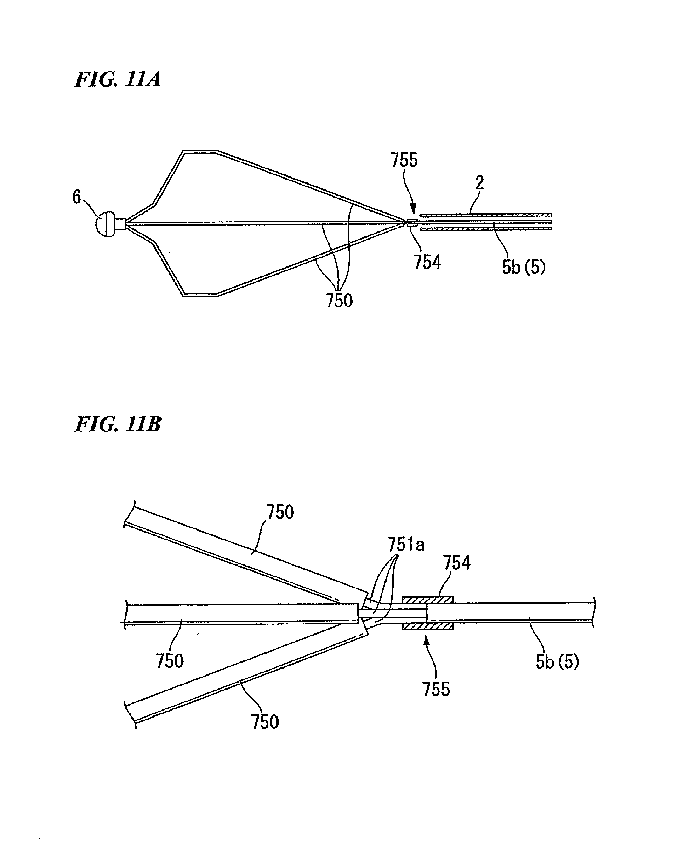

Next, a treatment instrument of a fifth embodiment of the present invention is described with reference to Fig. 11A and Fig. 11B. In the present embodiment, wires 750 are multiply provided in which small-diameter parts are formed similar to those of the wire 350 of the first embodiment. As shown in Fig. 11A, the end parts of the multiple wires 750 are respectively bundled at a first bundled part 6 and a second bundled part 755.
For example, Fig. 11B is an enlarged view which shows a partial cross-section in the vicinity of the second bundled part 755. As shown in Fig. 11B, small-diameter parts 751a provided at the end parts of the wires 750 are inserted into and fixed to a connecting tube 754. The small-diameter parts 751a are configured by reducing the diameter of the outer circumference of both ends of the wires 750 to a prescribed diameter. Although the details are not illustrated in the drawings, end parts of the wires including small-diameter parts are also similarly inserted into and fixed to the first bundled part 6.
A basket configuration similar to that of the third embodiment is obtained with this type of configuration as well. By bundling multiple wires in the same way as the third embodiment, it is possible to reduce the maximum external diameter of the portions where the wires are bundled in comparison to conventional baskets where the bundled portions are of large diameter.

While preferred embodiments of the invention have been described and illustrated above, it should be understood that these are exemplary of the invention, and are not to be considered as limiting. Additions, omissions, substitutions, and other modifications can be made without departing from the spirit or scope of the present invention. Accordingly, the invention is not to be considered as being limited by the foregoing description, and is only limited by the scope of the appended claims.

### [Industrial Applicability]

According to the treatment instrument of the present invention, it is possible to reduce the maximum external diameter of the connecting part of the bundled part and the manipulation wire, and create a treatment instrument of smaller diameter.

### [Description of the Referenced Symbols]

- 1:: treatment instrument
- 3:: treatment part
- 4:: manipulation part
- 5:: manipulation wire
- 55, 255, 455:: bundled part
- 6:: first bundled part
- 555, 655, 755:: second bundled part
- 46:: supply electrode
- 50, 250, 350, 450, 550, 650, 750:: wire
- 51, 251, 351, 451, 551, 651, 751:: first end part
- 52, 252, 352, 452, 552, 652, 752:: second end part
- 53, 253, 353, 453:: immediate part
- 54, 554, 654, 754:: connecting tube
- 51a, 52a, 551a, 552a:: small-diameter part
- 251a, 252a, 351a, 352a, 451a, 651a, 652a, 751a, 752a:: small-diameter part

## Claims

1. A treatment instrument, comprising:
a flexible wire that has a first end part and a second end part, and that has a treatment part at an intermediate part for conducting treatment within a body cavity;
small-diameter parts where at least a portion of the outer circumferential face of the wire has a smaller diameter or is denuded relative to the rest;
a bundled part where the wire is bundled so as to at least include the small-diameter parts; and
a manipulation wire that is connected to the bundled part.

2. The treatment instrument according to claim 1, wherein the small-diameter parts are provided at least on the first end part and the second end part.

3. The treatment instrument according to claim 1, comprising a connecting tube into which at least the small-diameter parts and the manipulation wire are inserted, and in which they are fixed.

4. A treatment instrument, comprising:
a flexible wire that has a first end part and a second end part, and that has a treatment part at an intermediate part for conducting treatment within a body cavity;
a small-diameter part where at least a portion of the outer circumferential face of the first end part has a smaller diameter or is denuded relative to the outer circumferential face of the intermediate part of the wire; and
a bundled part where the wire is curved or folded back, and the small-diameter part is fixed to the outer circumferential face of the wire;
wherein a length from the treatment part to the second end part is longer than a length from the treatment part to the first end part.

5. The treatment instrument according to claim 1, wherein the small-diameter parts have a tapered portion where a diameter narrows in a tapered form.

6. The treatment instrument according to claim 1, wherein the wire has electrical conductivity; and which further comprises conductive members that are electrically connected to the wire and that serve to supply high-frequency current.

7. The treatment instrument according to claim 6, wherein the manipulation wire has electrical conductivity.

8. The treatment instrument according to claim 6, wherein the treatment part is a snare wire that tightly binds body tissue and performs cautery incisions.

9. The treatment instrument according to claim 1, which has a plurality of the wires, and
wherein the treatment part provides with a basket that contacts the body tissue at three or more separate points relative to a substance within the body cavity, and that captures the substance within the body cavity.
